(19) Europäisches Patentamt
European Patent Office
Office européen des brevets

(11) Publication number: 0 075 895 A2

# (12) EUROPEAN PATENT APPLICATION

(21) Application number: 82108859.8

(22) Date of filing: 24.09.82

(51) Int. Cl.³: C 07 F 9/38
C 07 F 9/40, A 61 K 31/66

(30) Priority: 25.09.81 GB 8129054

(43) Date of publication of application: 06.04.83 Bulletin 83/14

(84) Designated Contracting States: AT BE CH DE FR GB IT LI LU NL SE

(71) Applicant: THE WELLCOME FOUNDATION LIMITED
183-193 Euston Road
London NW1 2BP(GB)

(72) Inventor: Wilkinson, Samuel
12 Bevington Road
Beckenham Kent(GB)

(72) Inventor: Hardy, George William
19 Alexandra Road
Biggin Hill Kent(GB)

(74) Representative: Berg, Wilhelm, Dr. et al,
Dr. Berg, Dipl.-Ing. Stapf, Dipl.-Ing. Schwabe, Dr. Dr. Sandmair Mauerkircherstrasse 45
D-8000 München 80(DE)

## (54) Pharmaceutical amides, and preparation, formulations and use thereof.

(57) Compounds of the general formula

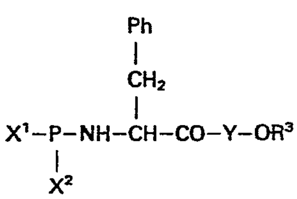

(I)

wherein $X^1$ is a group $R^1O$ and $X^2$ is a group $R^2O$, or one of $X^1$ and $X^2$ is a group $R^4$ and the other is hydroxy, wherein, $R^1$, $R^2$, $R^3$ and $R^4$ are the same or different and each represent hydrogen or a $C_{1-4}$ alkyl group;

Ph is a phenyl group, optionally substituted by one or more groups independently selected from halo nitro; and

Y is a group of formula

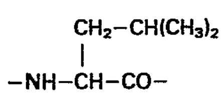

or a group of formula

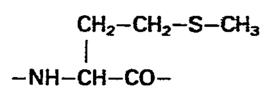

or a group of formula

$-NH-CH_2-CO-$;

and basic salts thereof. These compounds have an advantageous enkephalinase inhibitory activity which renders the compounds useful in medical therapy, eg. to prolong and/or potentiate in a mammal, the effects of endogenous or exogenous enkephalins. The latter includes synthetic enkephalin analogues.

EP 0 075 895 A2

Pharmaceutical amides, and preparation, formulations and use thereof

This invention relates to amides and their preparation, to pharmaceutical formulations containing such compounds and the preparation of such formulations, to the use of such compounds in human and veterinary medicine, and to intermediates of value in the preparation of the amides and the preparation of such intermediates.

In 1975, Hughes et al., (Nature Vol. 258, December 18, 1975 pages 577 to 579) identified two related pentapeptides from the mammalian brain with potent opiate agonist activity, the enkephalins:

H.Tyr.Gly.Gly.Phe.Met.OH(Met$^5$-enkephalin)

H.Tyr.Gly.Gly.Phe.Leu.OH(Leu$^5$-enkephalin)

(The abbreviations used herein for amino acids and their radicals are those conventional in the art and may be found in, for example, Biochemical Journal (1972) 126, pages 773 to 780. In the above and throughout the following all references are to the L-configuration of chiral amino acids and their radicals unless otherwise stated).

Since the discovery the enkephalins have been studied by a number of workers and from a variety of approaches. One such approach concerns investigation of their inactivation and recent reports (for example Malfroy et al., Nature Vol. 276, 30 November 1978 pages 523 to 526 and Gorenstein et al., Life Sciences Vol. 25 (1979) pages 2065 to 2070) have indicated that there exists in mammalian brain a dipeptidlcarboxypeptidase ("enkephalinase") capable of hydrolysing the Gly$^3$-Phe$^4$ bond

H.Tyr.Gly.Gly.Phe.$^{\text{Met}}_{\text{Leu}}$.OH

and thus generating the biologically inactive N-terminal tripeptide fragment

H.Tyr.Gly.Gly.OH

Enkephalinase thus has a role in some ways comparable with that of the mammalian angiotensin converting enzyme (ACE, EC 3.4.15.1) which acts upon the relatively inactive decapeptide angiotensin I

H.Asp.Arg.Val.Tyr.$^{\text{Val}}_{\text{Ile}}$.His.Pro.Phe.His.Leu.OH

at the Phe$^8$-His$^9$ bond to release the potent pressor octapeptide angiotensin II.

H.Asp.Arg.Val.Tyr.$^{\text{Val}}_{\text{Ile}}$.His.Pro.Phe.OH

RMW/JAH/14th September, 1982.

although it has been demonstrated (Swerts et al., European Journal of Pharmacology Vol. 57 (1979) pages 279 to 281) that the two enzymes are distinct species.

Controlling the liberation of angiotensin II from angiotensin I, by selectively inhibiting ACE, has for some time been regarded as a possible method for the therapy of hypertension and a number of agents, originating from such an approach and exhibiting the desired properties, have been described. One especially potent compound is 1-(D-3-mercapto-2-methylpropanoyl)-L-proline (S,S), otherwise known as Captopril or SQ 14 225 and having the structure

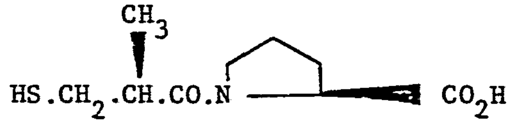

This has been reported as capable of inhibiting both enkephalinase and ACE (Swerts et al., loc. cit.) but as having a far greater specificity for the latter enzyme than for the former, the concentration of compound required to inhibit ACE by 50% being approximately 1000-fold lower than that required to effect the same degree of inhibition of enkephalinase.

Published Eurpean Patent Application EP 0 038 758 A1 (Roques, Schwartz and Lecomte) describes amino-acid derivatives, said to be capable of inhibiting enkephalinase, in particular, there is disclosed a compound 'Thiorphan' which has the formula

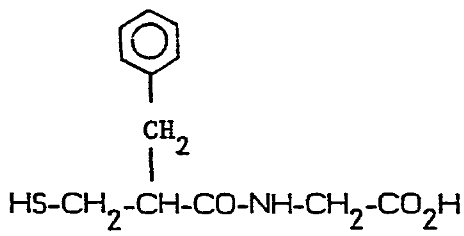

(DL)-3-mercapto-2-benzylpropanoyl)-glycine.

The present invention relates to a class of compounds which have not only an advantageous enkephalinase inhibitory activity but also, in distinction to SQ 14 225, a greater specificity for enkephalinase than for ACE.

The present invention thus provides the amides of formula

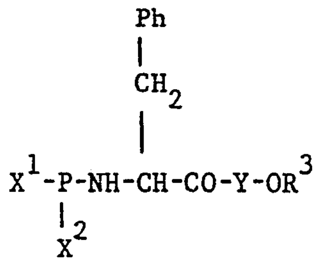

(I)

together with basic salts thereof (i.e. salts formed by reaction of a compound of formula (I) with a base) wherein:

$X^1$ is a group $R^1O$ and $X^2$ is a group $R^2O$, or one of $X^1$ and $X^2$ is a group $R^4$ and the other is hydroxy, wherein, $R^1$, $R^2$ $R^3$ and $R^4$ may be the same or different and each represent hydrogen or a $C_{1-4}$alkyl group i.e., methyl, ethyl, n-propyl, isopropyl, n-butyl, isobutyl, or t-butyl;

Ph is phenyl group which is optionally substituted by one or more substitutents selected from halo (i.e. fluoro, chloro, bromo, or iodo) and nitro; and Y is a group of formula

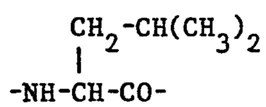 or 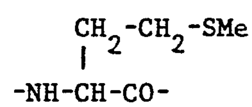 or 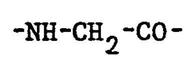

Formula (I) as above defined includes a plurality of asymmetric centres and should be understood to include all optical isomers embraced thereby and mixtures thereof.

For compounds of formula (I) as defined above, wherein one of $R^1$ and $R^2$ is a $C_{1-4}$alkyl group, the other of $R^1$ and $R^2$ may desirably be hydrogen.

In the salts of the amides of formula (I) the pharmacological activity resides in the amide (acid) anion and the identity of the cation is of less importance although for therapeutic purposes it is preferably pharmacologically and pharmaceutically acceptable to the recipient. Acceptable salts include ammonium salts, alkali metal salts such as sodium and potassium salts, alkaline earth metal salts such as magnesium and calcium salts, and salts of organic bases, for example amine salts derived from mono-, di- or trilower alkylamines or cycloalkylamines

such as dicyclohexylamine or alkanolamines such as triethanolamine and diethylaminoethylamine and salts with heterocyclic amines such as pipiridine, pyridine, piperazine and morpholine.

As subclasses of the amides of formula (I), may be mentioned, (including any appropriate combination thereof) those lised below, as well as processes for their preparation, formulations containing them, methods of treatment (eg of a mammal) by their administration, or said amides for use in prophylaxis and/or treatment of a mammal. Such processes, formulations, methods of treatment and uses, may for example, be any of those described hereinafter. These subclasses are those of the amides of formula (I) wherein:

(i) $X^1$ is a group $R^1O$ and $X^2$ is a group $R^2O$ ($R^1$ and $R^2$ being as defined above), Ph is a phenyl group optionally substituted by one or more groups independently selected from halo and nitro, and Y is a group of formula

$$\begin{array}{l} \quad\;\; CH_2\text{-}CH(CH_3)_2 \\ \quad\;\; | \\ \text{-}NH\text{-}CH\text{-}CO\text{-} \end{array}$$

or a group of formula

$$\begin{array}{l} \quad\;\; CH_2\text{-}CH_2\text{-}S\text{-}CH_3 \\ \quad\;\; | \\ \text{-}NH\text{-}CH\text{-}CO\text{-}; \end{array}$$

(ii) one of $X^1$ and $X^2$ is a group $R^4$ (as defined above) and the other is hydroxy, and/or Y is a group of formula $-NH-CH_2-CO-$;

(iii) $R^3$ is hydrogen;

(iv) $R^3$ is a methyl, ethyl, n-propyl, isopropyl, n-butyl, isobutyl, or t-butyl group, in particular a methyl, ethyl or t-butyl group;

(v) one of $R^1$ and $R^2$ is a $C_{1-4}$alkyl group and the other of $R^1$ and $R^2$ is hydrogen;

(vi) Y is a group of formula

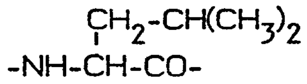

(in either the D- or L- configuration);

(vii) Y is a group of formula

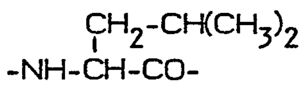

(in either the D- or L- configuration)

(viii) Y is a group of formula $-NH-CH_2-CO-$.

The amides of formula (I) and their basic salts may be prepared by any of the methods known in the art for the preparation of compounds of analogous structure. Thus they may be prepared by reacting a compound of formula

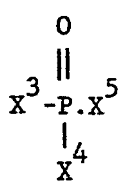

(II)

(wherein $X^3$ is a group $R^1a$ and $X^4$ is a group $R^2a$, in which $R^1a$ and $R^2a$ may be the same or different and each represent a hydroxy protecting group, e.g. an aralkyl group such as p-nitrobenzyl, or one of $X^3$ and $X^4$ is a group $R^4$ as defined above, and the other is hydroxy; and $X^5$ is a displaceable radical such as halo, e.g. chloro) with a compound of formula

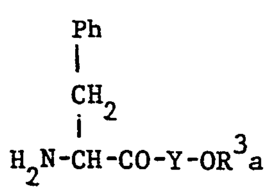

(III)

(wherein Ph and Y are as defined above and $R^3a$ is a group $R^3$ as defined in formula (I) or a functionally protected derivative thereof) followed, as appropriate, by deprotection of the product and conversion into an amide of formula (I) or a basic salt thereof.

The starting materials of formula (II) may be prepared in conventional manner, e.g. in an analogous manner to that described by L.Zervas and I.Dilaris J.A.C.S., 1955, 77, 5354; and Chem. Ber., 1956, 89, 925.

Deprotection in the above-described process may be effected in conventional manner, e.g. by hydrogenolysis using for example, palladium charcoal to remove athe aralkyl $R^1a$ and $R^2a$ protecting groups.

Certain of the amides of formula (I) may also be prepared from precursors which are themselves within formula (I).

Thus,

(i) compounds wherein $R^3$ is hydrogen may be prepared by hydrolysis of corresponding compound where $R^3$ is $C_{1-4}$alkyl;

(ii) compounds wherein $R^3$ is $C_{1-4}$alkyl may be prepared by esterification of the corresponding compound where $R^3$ is hydrogen.

The amides of formula (I) may be converted into basic salts thereof, and the converse, by well established techniques.

When the preparative procedures hereinabove described provide a mixture of optical isomers of the amide of formula (I) or of an intermediate thereto, for example a mixture of diastereoisomers, the individual isomers may be separated by appropriate conventional physical techniques such as high performance liquid chromatography, preparative thin layer chromatograph and the like.

Because of their selective enkephalinase-inhibiting activity the amides of formula (I) and the basic salts thereof are of value in the in vitro and in vivo investigation of the mode of action and the role of the enzyme and in its localization, isolation and purification.

For example, the present invention provides a method which comprises contacting an amide of formula (I) or a basic salt thereof with enkephalinase and determining the inhibitory effect of the amide or salt on the enkephalinase and determining the inhibitory effect of the amide or salt on the enzyme activity of the

enkephalinase. This method can be used to compare the enkephalinase inhibitory effect of the above compounds with other compounds having a similar effect. The compounds according to the invention can be radiolabelled, if desired, to facilitate the determination of their inhibitory effect.

Their selective enkephalinase-inhibiting activity also confers on the amides of formula (I) and the pharmacologically and pharmaceutically acceptable basic salts thereof utility in the prolongation and/or potentiation in a mammal of the effects of enkephalins of either endogenous or exogenous origin including in the later case synthetic enkephalin analogues. The said amides and salts thus have the same activities and uitlities as have been indicated for the endogenous compounds.

The amides of formula (I) may therefore be useful for prophylaxis and/or treatment of anxiety in mammals such as man, e.g. by virtue of an ability to induce tranquillisation.

The amides of formula (I) may also be useful for prophylaxis and/or treatment of convulsions in mammals such as man.

A recent study (G.E. Sander et al, Peptides Vol. 2 (1981) pp 403-407) has suggested a role for enkephalins in cardio-pulmonary function. The amides of formula (I) may therefore also be useful for the prophylaxis and/or treatment of a condition of a mammal such as man, wherein an improvement in cardio-pulmonary function is indicated, for example to relieve dyspnoea, e.g. that of acute left ventricular failure or pulmonary oedema.

Furthermore, the amides of formula (I) and the pharmacologically and pharmaceutically acceptable basic salts thereof have morphinomimetic (morphine agonist) activity and thus may be used in the treatment of mammals in the fields of both human and veterinary medicine in any condition where an agent with a morphine-like effect is indicated.

The pharmacological properties and therapeutic uses of morphine are well documented in the literature (see for example 'The Pharmacological Basis of Therapeutics', Goodman, LS and Gilman, A eds., published by Macmillan Publishing Co., Inc., New York, fifth edition (1975), ISBN 0-02-344781-8, especially at Chapter 15 pages 245 to 283, and 'Martindale: The Extra Pharmacopoeia', Wade, A ed., published by The Pharmaceutical Press, London, twenty-seventh edition (1977), ISBN 0-85369-114-2, especially at pages 970 to 974) and specific utilities for

the said amides and salts include, by way of example, the following.

(1) The relief of pain (analgesia), for example pain arising from spasm of smooth muscle as in renal or biliary colic, pain in terminal illness such as terminal cancer,

pain in the postoperative period, and obstetrical pain.

(2) The induction of constipation, for example after ileostomy or colostomy.

(3) The treatment of diarrhoea or dysentery.

(4) The suppression of cough.

(5) The induction of sleep, especially where sleeplessness is due to pain or cough.

(6) Sedation, for example in pre-anaesthetic medication to reduce preoperative apprehension.

(7) The induction of euphoria and the treatment of depression, for example when allied to the relief of pain in terminal illness such as terminal cancer.

The amides of formula (I) and the pharmacologically and pharmaceutically acceptable basic salts thereof (hereafter collectively referred to as the active ingredients) may be administered to the human or non-human recipient by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general however, for each of these utilities and indications, a suitable, effective dose will be in the range 0.075µg to 12mg per kilogram bodyweight per day, preferably in the range 0.75mg to 1.2mg per kilogram bodyweight and most preferably in the range 7.5 to 120µg per kilogram bodyweight per day, an optimum dose is 30mg per kilogram bodyweight per day. (Unless otherwise indicated all weights of active ingredient are calculated as the amide of formula (I): for salts thereof the figures would be increased proportionately). The desired dose is preferably presented as between two and four sub-doses administered at appropriate intervals throughout the day. Thus where three sub-doses are employed each will generally lie in the range 0.025µg to 4mg, preferably 0.25µg to 0.4mg and most preferably 2.5 to 40mg per kilogram bodyweight with an optimum of 10µg per kilogram bodyweight. A daily dose for a human weight of the order of 50kg will thus generally lie in the range 3.75µg to 600mg, in the range 37.5mg to 60mg and most preferably in the range 0.375 to 6.0 mg and may coneniently be presented as three equal unit sub-doses of 1.25µg to 200mg, preferably 12.5µg to 20mg and most

preferably 0.125 to 2.0mg. Optimally a human daily dose, for an individual weighing of the order of 50kg, is 1.5mg conveniently presented as three unite sub-doses each of 0.5mg.

While it is possible for the active ingredients to be administered as the raw chemical it is preferable to present them as a pharmaceutical formulation preparation. The formulations, both veterinary and for human use, of the present invention comprise an active ingredient, as above defined, together with one or more acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal intrathecal and epidural) administration. The formulations may conveniently be presented in unit dosage form and may be prepared by any of the methods well known in the art of pharmacy. All methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for rectal administration may be presented as a suppository with the usual carriers such as cocoa butter.

Formulations suitable for nasal administration wherein the carrier is a solid include a coarse powder having a particle size for example in the range 20 to 500 microns which is administrered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oil solutions of the active ingredient.

Formulations suitable for topical administration in the mouth include lozenges comprising the active ingredient in a flavoured basis, usually sucrose and acacia or tragacanth; and pastilles comprising the active ingredient in an inert basis such as gelatin and glycerin, or sucrose and acacia.

Formulations suitable for vaginal administration may be presented as pessaries, creams, pastes or spray formulations containing in additon to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspension which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously descirbed.

Preferred unit dosage formulations are those containing a daily dose or unit daily sub-dose, as hereinabove recited, or an appropriate fraction thereof, of an active ingredient.

It should be understood that in addition to the ingredients particularly mentioned above the formulation of this invention may include other agents conventional in the art having regard to the type of formulation in question, for example those suitable for oral administration may include flavouring agents.

All references identified hereinabove or in the following are hereby incorporated herein by reference thereto.

RMW/JAH/14th September, 1982.

Those basic salts which are not pharmacologically and pharmaceutically acceptable may be converted to the amides themselves and to salts thereof which are acceptable by standard procedures.

It will be understood from the foregoing description that this invention may comprise any novel feature described herein, principally but not exclusively for example:

(a) Amides of formula (I) as hereinbefore defined and the basic salts thereof.

(b) Methods as hereinbefore described for the preparation of compounds according to (a) supra, together with the compounds when so prepared.

(c) A pharmaceutical formulation comprising a therapeutically effective amount of an amide of formula (I) as hereinbefore defined or a pharmacologically and pharmaceutically acceptable basic salt thereof together with an acceptable carrier therefor.

(d) A method for the preparation of a formulation according to (c) supra comprising admixture of the active ingredient, as defined, with the carrier therefor.

(e) Amides of formula (I) as hereinbefore defined and pharmacologically and pharmaceutically acceptable basic salts thereof, for use in the therapeutic treatment of a mammal.

(f) Amides of formula (I) as hereinbefore defined and pharmacologically and pharmaceutically acceptable basic salts thereof, for use in the therapeutic treatment of a human.

(g) Amides of formula (I) as hereinbefore defined and pharmacologically and pharmaceutically acceptable basic salts thereof, for use in prolongation and/or potentiation in a mammal of the effects of endogenous or exogenous enkephalins.

(h) Amides of formula (I) as hereinbefore defined and pharmacologically and pharmaceutically acceptable basic salts thereof, for use in the prophylaxis and/or treatment of anxiety in a mammal such as man.

(i) Amides of formula (I) as hereinbefore defined, and pharmacologically and pharmaceutically acceptable basic salts thereof, for use in the prophylaxis and/or treatment of convulsions in a mammal such as man.

(j) Amides of formula (I) as hereinbefore defined and pharmacologically and pharmaceutically acceptable basic salts thereof, for use in the prophylaxis and/or treatment of a condition of a mammal such as man where an improvement in cardio-pulmonary function is indicated.

(k) Amides of formula (I) as hereinbefore defined and pharmacologically and pharmaceutically acceptable basic salts thereof, for the relief of dyspnoea in a

mammal such as man, e.g. that of acute left ventricular failure or pulmonary oedema.

(l) Amides of formula (I) as hereinbefore defined and pharmacologically and pharmaceutically acceptable basic salts thereof, for use in the treatment of a mammal for a condition where an agent with a morphine-like effect is indicated.

(m) Amides of formula (I) as hereinbefore defined and pharmacologically and pharmaceutically acceptable basic salts thereof, for use in the treatment of a mammal for a condition selected from those specifically identified hereinabove under (1), (2), (3), (4), (5), (6) or (7).

(n) A method for the prolongation and/or potentiation in a mammal of the effects of endogenous or exogenous enkephalins comprising administration to the mammal of an non-toxic, therapeutically effective amount of an amide of formula (I) as hereinbefore defined or a pharmacologically and pharmaceutically acceptable basic salt thereof.

(o) A method of prophylaxis and/or treatment of anxiety in a mammal, comprising administration to the mammal of a non-toxic, therapeutically effective amount of an amide of formula (I) as hereinbefore defined or a pharmacologically and pharmaceutically acceptable basic salt thereof.

(p) A method of prophylaxis and/or treatment of convulsions in a mammal, comprising administration to the mammal of a non-toxic, therapeutically effective amount of an amide fo formula (I) as hereinbefore defined or a pharmacologically and pharmaceutically acceptable basic salt thereof.

(q) A method of prophylaxis and/or treatment of a mammal for a condition where an improvement in cardio-pulmonary function is indicated, comprising administration to the mammal of a non-toxic, therapeutically effective amount of an amide of formula (I) as hereinbefore defined or a pharmacologically and pharmaceutically acceptable basic salt thereof.

(r) A method for the relief of dyspnoea (e.g. that of acute left ventricular failure or pulmonary oedema) in a mammal, comprising administration to the mammal of a non-toxic, therapeutically effective amount of an amide of formula (I) as hereinbefore defined or a pharmacologically and pharmaceutically acceptable basic salt thereof.

(s) A method for the treatment of a mammal for a condition where an agent with a morphine-like effect is indficated comprising administration to the mammal of a non-toxic, therapeutically effective amount of an amide of formula (I) as

hereinbefore defined or a pharmacologically and pharmaceutically acceptable basic salt thereof.

(t) A method for the treatment of a mammal for a condition selected from those specifically identified hereinabove under (1), (2) (3), (4), (5), (6) or (7) comprising administration to the mammal of a non-toxic, therapeutically effective amount of an amide of formula (I) as hereinbefore defined or a pharmacologically and pharmaceutically acceptable basic salt thereof.

(u) A method according to (n), (o), (p), (q), (r), (s) or (t) supra wherein the mammal is man.

The following Example is provided in illustration of the present invention and should not be construed as in any way constituting a limitation thereof. All temperatures are in degrees Celsius.

Abbreviations

Z = Benzyloxycarbonyl
Pd-C = palladium charcoal

Example 1 N-(Phosphoryl-phenylalanyl)leucine (Compound No. 1)

(a) Bis-p-nitrobenzyl phosphonate

A mixture of tri-p-nitrobenzyl phosphate (6g) and NaI (2g) in acetone (20ml) was refluxed for 15 min. On cooling the crystalline sodium salt was collected, washed with acetone and dried (4.35g).

The free acid was obtained by acidifying an aqueous solution of the sodium salt with 2M.HC1(10ml). The precipitated solid (4.2g) was filtered and dried.

(b) Bis-p-nitrobenzylphosphoryl chloride

The product of stage (a) (4.0g) was suspended in $CHC1_3$ (20ml) and stirred at $0^oC$ whilst $PC1_5$(2.48g) was added in three portions. After stirring at ambient temperature overnight, the suspension was filtered. Light petroleum (b.p. 40-60$^o$) was added to the filtrate and the crystalline precipitate filtered, washed with light petroleum and dried in vacuo, (2.82g) m.p. 106-107$^o$.

(c) N-(Bis-p-nitrobenzyl)phosphoryl-phenylalanyl-leucine benzyl ester

To a solution of Phe.Leu.OBzl.HCl(2.03g) and $NEt_3$ (1.1g) in $CHCl_3$(20ml) cooled to $0^oC$ was added the product of stage (b) (1.93g). The mixture was stirred at ambient temperature overnight. The product was evaporated in vacuo and the residue partitioned between Et OAc (100ml) and $H_2O$(50ml). The EtOAc phase was acid/base washed in the normal manner, dried ($MgSO_4$) and concentrated in vacuo. The solid residue was crystallised from ethanol (2.75g, m.p. $123^oC$).

| | | |
|---|---|---|
| $C_{36}H_{39}N_4O_{10}P$ | Requires: | C, 60.16; H, 5.47; N, 7.80 |
| | Found: | C,60.30; H, 5.47; N, 7.73% |

(d) N-(Phosphoryl-phenylalanyl)-leucine

The product of stage (c) (0.5g) was hydrogenated in 50% aqueous methanol (25ml) in the presence of $NaHCO_3$(175mg) and 5% Pd.C (100mg). The theoretical amount of hydrogen was taken up in about 1 hr. The product was filtered, diluted

with water (25 ml) and partially evaporated in vacuo The aqueous solution was extracted with EtOAc (3 x 15 ml) and then lyophilised, to give the trisodium salt as a tan-coloured powder (330 mg).

| | | |
|---|---|---|
| $C_{15}H_{20}N_2O_6PNa_3,3H_2O$ | requires: | C, 37.66; H, 5.47; N, 5.85 |
| | Found: | C, 37.60; H, 5.20; N, 5.37% |

Example 2 N-Phosphoryl-L-phenylalanyl-glycine trisodium salt
(Compound 2)

(a) t-Butyloxycarbonyl-L-phenylalanylglycine methyl ester

Glycine methyl ester hydrochloride (B.D.H., 4.73 g; 37.7 mmol) 1-hydroxybenzotriazole hydrate (1.0 g; 6.53 mmol) and t-butyloxycarbonyl -L-phenylalanine (prepared according to the prodcedure of Houben-Weyl 'Methoden der Organischen Chemie' Vol. 15 part 1. p.131.) were dissolved in dichloromethane (50 ml) and cooled to $0^oC$. Ethyl diisopropyl amine (Aldrich, 6.6 ml; 37.7 mmol) and dicyclohexylcarbodiimide (7.77 g; 37.7 mmol) were added with stirring. The mixture was allowed to warm to room temperature over night (16 hr).
Dicyclohexylurea was removed by filtration and the filtrate evaporated. The residue in ethyl acetate ( 100 ml) was filtered and washed with water (2 x 50 ml), saturated $NaHCO_3$ (2 x 50 ml), 0.7 M citric acid (2 x 50 ml), water

(50 ml), brine (100 ml) and dried over $Na_2SO_4$. The residue after evaporation was crystallised from ethyl acetate ( 20 ml) - petrol ether (b.p. 60-80$^oC$ added to cloud point). After 10 days at 4$^oC$ the product was collected.

Yielded 10.93 g (86%) m.p. 94-95$^oC$. Homoegeneous on tlc; chloroform-ethanol (95:5) Rf 0.69. Chloroform - methanol - 32% aq. AcOH (120:90:5) Rf. 0.81. amino acid analysis : Phe 1.00, Gly 1.01.

(b) L-Phenylalanylglycine methyl ester hydrochloride

t-Butyloxycarbonyl-L-phenylalanylglycine methyl ester (1.5 g 4.4 mmol) was stirred for 30 min. with 1M HCl-AcOH (15 ml) then evaporated. The hygroscopic solid obtained by repeated trituration of the residue with dry ether was dried in vacuo over $P_2O_5$ and used directly below.

Yield 1.25 g. Homogeneous on the tlc $CHCl_3$-MeOH-32% aq. AcOH (120:90:5) Rf 0.54.

(c) N-[Bis-4-nitrobenzylphosphoryl]-L-phenylalanylglycine methyl ester

Phenylalanylglycine methyl ester hydrochloride (1.2 g; 4.4 mmol) was dissolved in dry $CHCl_3$ (70 ml), triethylamine (0.98 g, 9.7 mmol) added and the stirred solution cooled to 0$^oC$. Bis-4-nitrobenzyl-phosphoryl chloride (Example 1b) (1.5 g, 3.9 mmol) was added and the mixture stirred for 17 h being allowed to warm to ambient temperature. The solution was washed with water (50 ml), 1M HCl (50 ml) - a white solid precipitated and was removed by filtration - washing was continued with 1M HCl (2 x 50 ml), saturated $NaHCO_3$ (3 x 50 ml) water (2 x 50 ml), brine (50 ml) and dried over $MgSO_4$. After evaporation the residue was crystallised from methanol-water. Yield 0.7 g (two crops) m.p. 143-145$^oC$. (31%) $C_{26}H_{27}N_4O_{10}P$ requires C 53.25, H 4.64, N 9.55 found C 53.57, H 4.93, N 9.54%. Homogeneous on tlc, methyl ethyl ketone. Rf 0.39.

(d) N-Phosphoryl-L-phenylalanyl-glycine trisodium salt

RMW/JAH/14th September, 1982.

N-(Bis-4-nitrobenzylphosphoryl)phenylalanylglycine methyl ester (0.49 g 0.84 mmol) was dissolved in dioxane (100 ml) and a solution of sodium hydroxide (0.1 g 2.5 mmol) in water (30 ml) added. The solution was stirred at ambient temperature for 17 h. 5% Palladium on charcoal (0.1 g) was added with a little water and the mixture stirred under hydrogen for 3 h. (Uptake 140 ml 93% theory, no further uptake). The catalyst was removed by filtration through celite under nitrogen. After evaporation the residue was dissolved in water and washed with ether (3 x) to remove p-toluidine. The aqueous phase was lyophilised, the residue redissolved in water and lyophilised again. The product was collected with the aid of dry ether and dried in high vacuum over $P_2O_5$. Yield 0.232g (75%) m.p. 300°C.

| | | |
|---|---|---|
| $C_{11}H_{12}N_2O_6PNa_3$. 0.5 $H_2O$ | requires | C 35.03, H 3.47, N 7.43 |
| | found: | C 35.25, H 3.81, N 7.02.% |

Example 3 N-Methylphosphinico-L-phenylalanyl-L-leucine disodium salt (Compound No. 3)

(a) Methyl methoxymethylphosphinyl-L-phenylalanyl-L-leucinate

L-Phenylalanyl-L-leucine methyl ester hydrochloride (1.0g; 3.04mmol) was suspended in chloroform (25ml dried over $CaCl_2$) at 0°C, triethylamine (0.65g; 6.4mmol) and methoxymethylphosphinic chloride (prepared according to the method of Z. Pelchowicz J. Chem. Soc. 1961, 238).

($CH_3$P-OMe, 0.42g; 3.3mmol) were added and the clear solution allowed to warm to ambient temperature overnight. Tlc indicated only partial reaction so a second equivalent of acid chloride and triethylamine were added (at0°) and the mixture left overnight. The solution was washed with 10ml portions of water (1 x), 0.7M citric acid (3 x), water (1 x), saturated $NaHCO_3$ (3 x), water (1 x), brine (1 x) and dried over $MgSO_4$. Evaporation left a pale yellow oil (1.26g) which spontaneously crystallised. The solid was collected after trituration with ether-hexane (1:4, 15ml). Yield 0.75g (64%) m.p. (106) 110-112°C.

RMW/JAH/14th September, 1982.

$C_{18}H_{29}N_2O_5P$ requires C 56.24, H 7.60, N 7.29
found: C 56.80, H 7.58, N 7.09.

Homogeneous in tlc: chloroform-methanol-32% aq. AcOH (120:90:5) Rf 0.88 N.m.r. spectrum consistant with proposed structure.

(b) Benzyl Methoxy methylphosphinyl-L-phenylalanyl-L-leucinate

Prepared in an analogous manner to the methyl ester with one equivalent of acid chloride on a 0.86mmol scale.

Yield 0.35g (88%) given which slowly crystallised. N.m.r. spectrum consistent with proposed structure.

(c) Methoxy methylphosphinyl-L-phenylalanyl-L-leucine

To the above benzyl ester (0.35g, 0.76mmol) was added a suspension of 5% palladium on charcoal (0.1g) in methanol-water (9:1, 50ml). The mixture was stirred under hydrogen for 15min when uptake ceased. (Uptake 15ml, theory 16ml). The suspension was filtered through celite and evaporated. The residue crystallised from ethyl acetate. Yield 178mg m.p. 186-188$^{\circ}$C.

$C_{17}H_{27}N_2O_5P.0.5\ H_2O$ requires: C 53.81, H 7.43, N 7.38
found: C 54.04, H 7.10, N 7.28%

(d) N-Methylphosphinico-L-phenylalanyl-L-leucine disodium salt

Methyl methoxymethylphosphinyl-L-phenylalanyl-L-leucinate (0.2g 0.52mmol) was dissolved in $CH_3CN$(1ml) and 1M NaOH (1.04ml; 1.04mmol) added. The solution was stirred overnight (17h) at room temperature then evaporated. The crystalline residue was triturated with acetone and ether and collected. Yield 148mg (71%) m.p.>320$^{\circ}$C.

$C_{16}H_{24}N_2O_5PNa_2.0.5H_2O$ requires: C 46.83, H 6.14, N 6.82
found: C 46.78, H 6.23, N 6.86%

RMW/JAH/14th September, 1982.

Alternatively the above disodium salt was prepared by the same methodology, but applied to the saponification of methoxy methylphosphinyl-L-phenylalanyl-L-leucine (part c above).

ACTIVITY IN VITRO

The compounds of the above Examples were investigated for enkephalinase-inhibiting activity using the following method.

Purified enkephalinase A1 was obtained according to the following procedure (modification of the method of Gorenstein and Snyder, Life Science, Vol. 25, pages 2065-2070, 1979).

Rats were killed by decapitation and the striata dissected out on ice. The pooled tissues were homogenised in ice-cold Tris/hydrochloric acid buffer (50mM, pH 7.70, 30ml per gram of tissue) and centrifuged (50,000g, 15 mins). The resulting supernatent was discarded and the remaining pellet washed three times. The washed pellet was solubilised by resuspension in half the volume of buffer as before containing 1.0% (v/v) Triton X-100 and incubated at 37°C for 45 mins. The suspension was then centrifuged at 100,000g for 60 mins and the solubilised enzymes contained in the supernatent separated by DEAE-cellulose column chromatograph. Enkephalinasse A1 was futher purified by Sephacryl S.300 chromatography.

Enkephalinase-inhibiting activity was estimated by the following procedure. 1.75µl of leucine enkephalin (0.317mg/ml), 0.5µl of $^3$H-leucine enkephalin (tyrosyl-3,5-$^3$H Enkephalin (5-L-leucine), The Radiochemical Centre, Amersham, England) and 5.75µl of buffer as before were incubated at 30°C for 10 mins with 2µl of either a solution of test compound (in either 50% ethanol/0.1M sodium hydrogen carbonatge or distilled water) or solvent alone as control. 10µl of purified enkephalinase A1 at 30°C were added and incubation then continued for a further 30 mins (total incubation time 40 mins, final leucine enkephalin concentration 5 x $10^{-5}$M, final $^3$H-leucine enkephalin concentration 12.5Ci/ml). At the completion of incubation 3µl of 0.16M hydrochloric acid was added and the incubation mixture cooled on ice.

Separation of (a) unchanged $^3$H-leucine enkephalin and (b) $^3$H-Try-Gly-Gly-OH ($^3$H-TGG), generat ed from (a) by enkephalinase A1 in the incubation mixture was

effected by thin layer chromatography (plastic silica gel plates of 0.1mm layer thickness, solvent system ethyl acetate: propan-2-ol: 5% (v/v) acetic acid, 2:2:1) using solutions of the cold compounds as carriers. After drying the materials were visualized with ninhydrin and appropriate areas of the plates cut out and placed in scintillation vials containing 50% methanol/0.1M hydrochloric acid to elute the $^3H$ label. Biofluor reagent (10ml) was then added and the radioactivity determined by liquid scintillation counting.

The $^3H$-TGG generated in the presence of test compound (expressed as a percentage of the control figure) was then calculated and an approximate $IC_{50}$ figure (concentration of test compound required for 50% inhibition of $^3H$-TGG generation) then determined graphically. For comparison, the $IC_{50}$ for Captopril, Thiorphan, and for the intermediate product of Example 4(c), methoxy methylphosphinyl-L-phenylalanyl-L-leucine (MMPL), are also given:

| Compound No. | $IC_{50}$(M) |
|---|---|
| 1 | $7 \times 10^{-9}$ |
| 2 | $1.5 \times 10^{-8}$ |
| 3 | $3.2 \times 10^{-8}$ |
| Captopril | $3 \times 10^{-3}$ |
| Thiorphan | $1.2 \times 10^{-8}$ |
| MMPL | 10% inhibition @ $10^{-4}$M |

RMW/JAH/14th September, 1982.

## Pharmaceutical Formulations

The compound of formula (I) employed in the following Examples of pharmaceutical formulations may be any compound of formula (I) defined above or a basic salt thereof.

### A) Tablet Formulation (0.5mg/tablet)

| | |
|---|---|
| Compound of formula (I) | 0.5mg |
| Maize Starch | 10mg |
| Polyvinylpyrrolidone | 2mg |
| Magnesium Stearate | 2mg |
| Lactose | to 100mg |

Mix together the compound of formula (I), Lactose and Maize Starch. Granulate with a solution of the Polyvinylpyrrolidone dissolved in water. Dry the granules, add the Magnesium Sterate and compress to produce tablets, 100mg per tablet.

### B) Suppository (0.5mg/product)

| | |
|---|---|
| Compound of formula (I) | 25mg |
| Suppository Base (Massa Esterinum C) | to 100 mg |

Melt the suppository base at 40$^{o}$C. Gradually incorporate the compound of formula (I) in fine powder form powder form and mix until homogenous. Pour into suitable moulds, 2g per mould, and allow to set. Massa Esterinum C is a commercially available suppository base consisting of a mixture of mono-, di-, and tri-glycerides of saturated vegetable fatty acids. It is marketed by Henkel International,Dusseldorf.

RMW/JAH/14th September, 1982.

C) Pessary (0.5mg/product)

| | |
|---|---|
| Compound of formula (I) | 0.5mg |
| Lactose | 400mg |
| Polyvinylpyrrolidone | 5mg |
| Magnesium Sterate | 4.5mg |

Mix together the compound of formula (I) and Lactose. Granulate with a solution of Polyvinylpyrrolidone in 50% aqueous ethanol. Dry the granules, add the Magnesium Stearate and compress on suitably shaped punches, 410mg per pessary.

D) Freeze-dried Injection 0.5mg/vial

| | |
|---|---|
| Compound of formula (I) | 0.5mg |
| Mannitol | 99.5mg |
| Water for Injections to | 2.0ml |

Dissolve the compound of formula (I) and mannitol in the Water for Injections. Sterilise the solution by passage through a membrane filter, 0.2μm pore size, collecting the filtrate in a sterile receiver. Fill into sterile glass vials, 2ml/vial under aseptic conditions and freeze-dry. Close the vials with sterile rubber closures secured with an aluminium seal.

The injection is reconstituted prior to administration by the addition of a convenient volume of Water for Injections or sterile saline solution.

RMW/JAH/14th September, 1982.

Claims:-

1) Compounds of the general formula

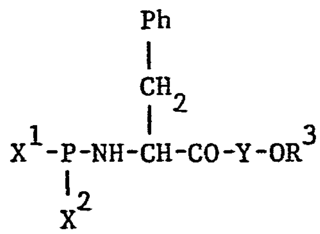

(I)

wherein $X^1$ is a group $R^1O$ and $X^2$ is a group $R^2O$, or one of $X^1$ and $X^2$ is a group $R^4$ and the other is hydroxy, wherein,

$R^1$, $R^2$, $R^3$ and $R^4$ are the same or different and each represent hydrogen or a $C_{1-4}$alkyl group;

Ph is a phenyl group, optionally substituted by one or more groups independently selected from halo and nitro; and

Y is a group of formula

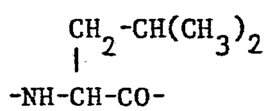

or a group of formula

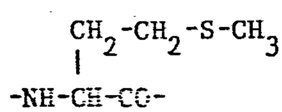

or a group of formula

$$-NH-CH_2-CO-$$

and basic salts thereof.

2) Compounds as claimed in claim 1, wherein $X^1$ is a group $R^1O$ and $X^2$ is a group $R^2O$($R_1$ and $R_2$ being as defined in claim 1), Ph is a phenyl group, optionally substituted by one or more groups independently selected from halo and nitro, and Y is a group of formula

$$\begin{array}{l} \quad\; CH_2\text{-}CH(CH_3)_2 \\ \quad\;\, | \\ \text{-}NH\text{-}CH\text{-}CO\text{-} \end{array}$$

(in either the D- or L- configuration),
or a group of formula

$$\begin{array}{l} \quad\; CH_2\text{-}CH_2\text{-}S\text{-}CH_3 \\ \quad\;\, | \\ \text{-}NH\text{-}CH\text{-}CO\text{-} \end{array}$$

(in either the D- or L- configuration).

3) Compounds as claimed in claim 1, wherein one of $X^1$ and $X^2$ is a group $R^4$ (as defined in claim 1) and the other is hydroxy, and/or Y is a group of formula $-NH-CH_2-CO-$;

4) Compounds as claimed in any of claims 1-3, wherein $R^3$ is hydrogen.

5) Compounds as claimed in any of claims 1-3, wherein $R^3$ is a methyl, ethyl, n-propyl, isopropyl, n-butyl, isobutyl, or t-butyl group.

6) Compounds as claimed in any of claims 1,2 or 4-6, wherein one of $R^1$ and $R^2$ is a $C_{1-4}$alkyl group and the other of $R^1$ and $R^2$ is hydrogen.

7) A compound selected from:-

N-(Phosphonyl-phenylalanyl)leucine and basic salts thereof;
N-(Phosphoryl-L-phenylalanyl)glycine and basic salts thereof;
N-(Phosphoryl-L-phenylalanyl)glycine trisodium salt;
N-(Methylphosphonico-L-phenylalanyl)-L-leucine and basic salts thereof; and
N-(Methylphosphonico-L-phenylalanyl)-L-leucine disodium salt.

8) A process for the preparation of compounds of formula (I)(as defined in claim 1) or a basic salt thereof, which comprises reacting a compound of formula

$$\begin{array}{c} O \\ \| \\ X^3\text{-}P.X^5 \\ | \\ X^4 \end{array} \qquad (II)$$

(wherein $X^3$ is a group $R^1a$ and $X^4$ is a group $R^2a$, in which $R^1a$ and $R^2a$ may be the same or different and each represent a hydroxy protecting group, eg. a an aralkyl group such as p-nitrobenzyl or one of $X^3$ and $X^4$ as defined above, and the other is hydroxy; and $X^5$ is a displaceable radical)

with a compound of formula

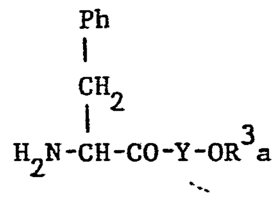
(III)

wherein, Ph and Y are as defined above, and $R^3a$ is a group $R^3$ as defined in claim 1 or a functionally protected derivative thereof;

optionally followed, as appropriate, by deprotection of the product and conversion into a compound of formula (I) or a basic salt thereof.

9) Pharmaceutical formulations comprising, as active ingredient, at least one compound of formula

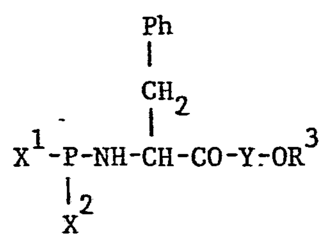
(I)

wherein $X^1$ is a group $R^1O$ and $X^2$ is a group $R^2O$, or one of $X^1$ and $X^2$ is a group $R^4$ and the other is hydroxy, wherein,

$R^i$, $R^2$, $R^3$ and $R^4$ are the same or different and each represent hydrogen or a $C_{1-4}$alkyl group;

Ph is a phenyl group optionally substituted by one or more groups independently selected from halo and nitro; and

Y is a group of formula

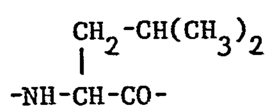

or a group of formula

$$\begin{array}{l} \quad CH_2-CH_2-S-CH_3 \\ \quad | \\ -NH-CH-CO- \end{array}$$

or a group of formula

$$-NH-CH_2-CO-$$

and physiologically acceptable basic salts thereof, together with one or more pharmaceutically acceptable carriers therefor and, optionally, one or more other therapeutic ingedients.

10) Compounds of formula (I), as defined in claim 1, and pharmacologically and pharmaceutically acceptable basic salts thereof, for use in the therapeutic treatment of a mammal such as man.

11) Compounds of formula (I), as defined in claim 1, and pharmacologically and pharmaceutically acceptable basic salts thereof, for use in the prolongation and/or potentiation in a mammal of the effects of endogenous or exogenous enkephalins.

12) Compounds of formula (I), as defined in claim 1, and pharmacologically and pharmaceutically acceptable basic salts thereof, for use in the prophylaxis and/or treatment of convulsions in a mammal.

13) Compounds of formula (I), as defined in claim 1, and pharmacologically and pharmaceutically acceptable basic salts thereof, for use in the prophylaxis and/or treatment of anxiety in a mammal.

14) Compounds of formula (I), as defined in claim 1, and pharmacologically and pharmaceutically acceptable basic salts thereof, for use in the prophylaxis and/or treatment of a condition of a mammal, where an improvement in cardio-pulmonary function is indicated.